## Europäisches Patentamt
## European Patent Office
## Office européen des brevets

(19)

(11) Veröffentlichungsnummer : **0 074 009**
**B1**

(12) EUROPÄISCHE PATENTSCHRIFT

(45) Veröffentlichungstag der Patentschrift :
01.10.86

(51) Int. Cl.⁴ : **C 07 D303/04, C 07 D301/04**

(21) Anmeldenummer : **82107726.0**

(22) Anmeldetag : **24.08.82**

(54) Verfahren zur Herstellung von Tetramethyloxiran.

(30) Priorität : 04.09.81 DE 3135112

(43) Veröffentlichungstag der Anmeldung :
16.03.83 Patentblatt 83/11

(45) Bekanntmachung des Hinweises auf die Patenterteilung : 01.10.86 Patentblatt 86/40

(84) Benannte Vertragsstaaten :
AT BE CH DE FR GB IT LI NL

(56) Entgegenhaltungen :
FR-A- 1 253 829
US-A- 3 153 058
US-A- 3 232 957
US-A- 3 238 229
Die Akte enthält technische Angaben, die nach dem
Eingang der Anmeldung eingereicht wurden und die
nicht in dieser Patentschrift enthalten sind.

(73) Patentinhaber : BAYER AG
Konzernverwaltung RP Patentabteilung
D-5090 Leverkusen 1 Bayerwerk (DE)

(72) Erfinder : Wilms, Klaus Günter, Dr.
Siegstrasse 3
D-4047 Dormagen 1 (DE)
Erfinder : Waldmann, Helmut, Dr.
H.-T.-v.-Böttinger-Strasse 15
D-5090 Leverkusen 1 (DE)
Erfinder : Grolig, Johann, Dr.
Heinrich-Lübke-Strasse 22
D-5090 Leverkusen 1 (DE)

**Beschreibung**

Die vorliegende Erfindung betrifft ein Verfahren zur Herstellung von Tetramethyloxiran (2,3-Dimethyl-2,3-epoxibutan) aus 2,3-Dimethylbutenen und Sauerstoff oder sauerstoffhaltigen Gasen.

Oxirane finden Verwendung auf dem Gebiet der Lacke, zur Herstellung von Polyethern, Polyurethanen, Epoxidharzen, Detergentien, Glykolen und als Zwischenprodukte (siehe z. B. US-PS 2 412 136).

Ethylenoxid, eine wichtige Verbindung aus der Gruppe der Oxirane, wird technisch durch Oxidation von Ethylen mit molekularem Sauerstoff in der Gasphase in Gegenwart von Silberkatalysatoren hergestellt (siehe beispielsweise US-PS 2 693 474). Eine Übertragung dieser Methode auf höhere Olefine scheiterte bislang, da dann in großem Ausmaß Nebenreaktionen ablaufen, welche die Wirtschaftlichkeit eines solchen Verfahrens entscheidend vermindern (siehe US-PS 2 412 136).

Es ist bekannt, Oxirane durch Cooxidation von Alkenen und Aldehyden mit Sauerstoff herzustellen. So kann man beispielsweise Tetramethyloxiran durch Cooxidation von 2,3-Dimethyl-2-buten und Acetaldehyd in Chlorbenzol als Lösungsmittel bei 40 °C in bis zu 84 %iger Ausbeute (gaschromatografisch ermittelt) darstellen (siehe H. Krapf und M.R. Yazdanbakch, Synthesis 1977, Seite 172, viertes Beispiel in der Tabelle).

Für die Wirtschaftlichkeit dieses Verfahrens ist es von großem Nachteil, daß pro Mol Oxiran ein Mol Essigsäure gebildet wird. Die Essigsäure muß z. B. durch Extraktion mit Natriumhydrogencarbonat-Lösung aus dem Reaktionsgemisch vor der weiteren Aufarbeitung entfernt werden, da das gebildete Tetramethyloxiran durch die Essigsäure gespalten und dadurch nur unter Verlusten isoliert werden kann (siehe I. Swern « Organic Peroxides », Vol. 2, Wiley Interscience, 1971, Seite 430, Zeile 9-11).

Ein weiteres Syntheseprinzip wird in den US-PSen 3 641 066, 4 256 649, 3 856 826 und 2 856 827 beschrieben. Dabei wird eine Flüssigphasenoxidation von Olefinen mit Sauerstoff oder Sauerstoff enthaltenden Inertgasen in Gegenwart von heterogenen Katalysatoren durchgeführt. Angaben über Umsatz und Selektivitäten können diesen Schriften nicht entnommen werden. Nachteilig ist aber immer die Verwendung von kostspieligen Katalysatoren und der Aufwand für deren Abtrennung, Aufarbeitung und Rückführung.

Eine weitere Methode zur katalytischen Olefin-Oxidation mit Sauerstoff wird in der US 4 182 722 beschrieben. Gemäß Beispiel B 2 wird die Oxidation von 2,3-Dimethyl-2-buten bei 50 °C und einer Reaktionsdauer von fünf Stunden in Gegenwart eines mit Kupfer$^{2+}$- und Vanadium$^{4+}$-Salzen ausgetauschten X-Zeolithen als Katalysator durchgeführt. Bei 24 % Olefin-Umsatz erhält man als Hauptprodukt 1,2-Epoxi-3-hydroxi-2,3-dimethylbutan mit einer Selektivität von 46 %. Als Nebenprodukte findet man Tetramethyloxiran mit 40 % Selektivität, 3-Hydroxi-2,3-dimethyl-1-buten mit 9 % Selektivität und Aceton mit 6 % Selektivität. Neben den schon erwähnten Nachteilen der Verwendung von Katalysatoren sind die lange Reaktionsdauer von fünf Stunden bei nur 24 % Olefin-Umsatz und die überwiegende Bildung von anderen Produkten als Tetramethyloxiran weitere Nachteile dieses Verfahrens.

Ein weiteres Verfahren zur Herstellung von Tetramethyloxiran aus Tetramethylethylen (2,3-Dimethyl-2-buten) betrifft den Einsatz eines homogenen Katalysatorsystems, das aus Komplexen der Formel trans-MCl(CO) (Ph$_3$P)$_2$ (M = Rhodium, Iridium ; Ph = Phenyl) besteht (siehe J.E. Lyons und J.O. Turner, J. Org. Chem., Vol. 37, 2881-2884 (1972)). Bei einer Reaktionsdauer von vier Stunden und einer Reaktionstemperatur von 50 °C erhält man bei einem Tetramethylethylen-Umsatz von 8 bis 66 % Tetramethyloxiran mit Selektivitäten von 25 bis 37,5 % (siehe Lit. Zitat : Seite 2882, Tabelle 1). Wegen der langen Reaktionszeit von vier Stunden und den sehr niedrigen Selektivitäten ist dieser Weg kein wirtschaftliches Verfahren zur Herstellung von Tetramethyloxiran.

Weiterhin ist bekannt, Tetramethylethylen bei 25 °C in Gegenwart von Eisen$^{3+}$-meso-tetraphenylporphyrinchlorid als Katalysator mit Sauerstoff umzusetzen (D.R. Paulson, R. Ullman, R.B. Sloane, J.C.S. Chem. Comm., 1974, Seite 187, Tabelle). Nach 24 Stunden Reaktionszeit erhält man als Hauptprodukt 2,3-Dimethyl-3-en-en-2-ol mit einer Selektivität von 45 % und als Nebenprodukte Tetramethyloxiran mit 41 % und Aceton mit 14 % Selektivität. Nachteile dieses Verfahrens sind neben den Katalysatorkosten die lange Reaktionszeit von 24 Stunden und die Bildung von 2,3-Dimethyl-3-en-2-ol als Hauptprodukt.

Außerdem ist es aus US-A 3 153 058 und US-A 3 232 957 bekannt, daß nur dann brauchbare Ergebnisse bei der Oxidation von Olefinen zu den entsprechenden Oxiranen erhalten werden, wenn in einer flüssigen Phase gearbeitet wird, die bestimmte Lösungsmittel enthält (Polyacylester von Polyhydroxyalkanen, Polyhydroxycycloalkanen und Polyglykolen einerseits oder Ketone andererseits). Es bestand deshalb keine Veranlassung, eine solche Oxidation ohne Zusatz von Lösungsmittel zu untersuchen.

Schließlich ist es bekannt, Tetramethyloxiran durch Sauerstoff-Oxidation von Tetramethylethylen bei 50 °C und 333 Minuten Reaktionszeit in Gegenwart von 2,2'-Azobis(2-methylpropionitril) herzustellen (I.E. Van Sickle, F.R. Mayo, R.M. Arluck, M. Syz. J. Am. Chem. Soc. 89, 1967, Seite 971, Tabelle 1). Dieses Verfahren ist wirtschaftlich nicht interessant, da der Tetramethylethylen-Umsatz trotz der über fünfstündigen Reaktionsdauer nur 5 % beträgt und die Tetramethyloxiran-Selektivität nur 15,5 % erreicht.

Es besteht also das Bedürfnis nach einem einfachen und effektiven Verfahren zur Herstellung von Tetramethyloxiran.

Es wurde nun ein Verfahren zur Herstellung von Tetramethyloxiran durch Umsetzung von 2,3-Dimethylbutenen, die bis zu 2 % 2,3-Dimethylbutan und bis zu 3 % n-Hexan enthalten können, mit Sauerstoff oder einem Sauerstoff enthaltenden Gas bei Temperaturen von 40 bis 180 °C und Verweilzeiten von 5 bis 150 Minuten ohne Zusatz von Katalysatoren gefunden, das dadurch gekennzeichnet ist, daß man ohne Zusatz von inerten Lösungsmitteln arbeitet und das erhaltene Tetramethyloxiran abtrennt.

Als in das erfindungsgemäße Verfahren einsetzbare 2,3-Dimethylbutene kommen beispielsweise 2,3-Dimethyl-2-buten, 2,3-Dimethyl-1-buten oder beliebige Gemische aus 2,3-Dimethyl-2-buten und 2,3-Dimethyl-1-buten in Frage.

2,3-Dimethyl-2-buten und 2,3-Dimethyl-1-buten sind leicht zugängliche Ausgangsstoffe, die man beispielsweise durch Dimerisierung von Propylen erhalten (siehe Houben-Weyl, Methoden der organischen Chemie, Band V/Ib, Seite 550 bis 551, Stuttgart (1972)) und dann sofort oder gegebenenfalls nach vorheriger Destillation in das erfindungsgemäße Verfahren einsetzen kann. Auch durch Dehydratisieren von 2,3-Dimethylbutan-2-ol können 2,3-Dimethylbutene erhalten werden, die in das erfindungsgemäße Verfahren eingesetzt werden können.

Vorzugsweise werden in das erfindungsgemäße Verfahren 2,3-Dimethyl-2-buten oder 2,3-Dimethyl-2-buten enthaltende Gemische der vorgenannten Art eingesetzt, die über 60 %, vorzugsweise über 70 % 2,3-Dimethyl-2-buten enthalten.

Der Sauerstoff kann dem erfindungsgemäßen Verfahren als reiner Sauerstoff oder in Form eines Sauerstoff enthaltenden Gasgemisches zugeführt werden. Wenn ein Sauerstoff enthaltendes Gasgemisch eingesetzt wird, enthält dieses neben Sauerstoff vorzugsweise nur noch inerte Bestandteile. Solche inerten Bestandteile können beispielsweise Stickstoff, Kohlendioxid und/oder Edelgase, wie Helium, Neon, Argon und/oder Krypton sein. Man kann auch ein, beispielsweise durch Verbrennung von Methan mit Luft herstellbares, Inertgasgemisch aus Kohlendioxid und Stickstoff als inerten Bestandteil des Sauerstoff enthaltenden Gasgemisches verwenden. Auch Zusätze von Ozon oder atomarem Sauerstoff sind möglich.

Vorzugsweise enthalten die Sauerstoff enthaltenden Gasgemische zwischen 10 und über 99 % Sauerstoff. Zum Einsatz in das erfindungsgemäße Verfahren verwendet man vorzugsweise Luft, Sauerstoff enthaltende Gasgemische, die über 50 % Sauerstoff enthalten oder reinen Sauerstoff.

Es kann vorteilhaft sein, den einzusetzenden Sauerstoff in mehreren Teilmengen nacheinander zuzugeben, beispielsweise indem man während der Umsetzung mehrmals Sauerstoff und/oder Sauerstoff enthaltende Gase aufdrückt. Wenn ein Sauerstoff enthaltendes Gasgemisch eingesetzt wird, können die verschiedenen Komponenten des Gasgemisches gemeinsam, getrennt oder in geeigneten Kombinationen zusammengefaßt der Reaktionszone zugeführt werden. Die Vermischung der Gaskomponenten kann innerhalb oder außerhalb der Reaktionszone erfolgen. Die Vermischung der Gaskomponenten außerhalb der Reaktionszone kann beispielsweise im Freistrahl, in Kanälen, in Mischapparaturen oder in Mischkammern erfolgen. Geeignet sind beispielsweise Injektormischer und Strahlmischer. Für die Gasvermischung können auch Rührorgane eingesetzt werden. Auch Gebläse können gleichzeitig als Mischvorrichtung dienen.

Es ist vorteilhaft, den Sauerstoffgehalt der Gasphase des Reaktionsraumes durch geeignete Maßnahmen so zu begrenzen, daß detonationsfähige Gasgemische nicht auftreten können. Solche Maßnahmen können z. B. bestehen in der Zugabe inerter Gase wie Stickstoff, Kohlendioxid und/oder Edelgasen.

Das erfindungsgemäße Verfahren wird bei Temperaturen von 40 bis 180 °C durchgeführt. Vorzugsweise arbeitet man bei 60 bis 160 °C, besonders bevorzugt bei 80 bis 140 °C.

Neben der Arbeitsweise unter isothermen Bedingungen, d. h. Einhaltung einer einheitlichen Temperatur während der gesamten Reaktion, kann man die Umsetzung auch unter Ausbildung eines sogenannten Temperaturgradienten durchführen, d. h. bei im Verlauf der Reaktion ansteigender oder abfallender Temperatur.

Das erfindungsgemäße Verfahren wird so durchgeführt, daß die Verweilzeit (bei diskontinuierlicher Arbeitsweise) bzw. die mittlere Verweilzeit (bei kontinuierlicher Arbeitsweise) 5 bis 150 Minuten beträgt. Vorzugsweise beträgt diese Zeit 10 bis 120 Minuten, besonders bevorzugt 15 bis 90 Minuten.

Das erfindungsgemäße Verfahren wird ohne den Zusatz von Katalysatoren durchgeführt.

Der Druck kann beim erfindungsgemäßen Verfahren in weiten Grenzen variieren. Es kann beispielsweise bei einem Systemdruck von 0,9 bis 90 bar durchgeführt werden. Vorzugsweise beträgt dieser Druck 2,0 bis 80 bar, besonders bevorzugt 5,0 bis 70 bar. In Sonderfällen können die angegebenen Druckbereiche auch unter- oder überschritten werden. Der Druck wird vorzugsweise eingestellt, indem man den gewünschten Reaktionsdruck durch Aufdrücken von z. B. Stickstoff, Kohlendioxid und/oder Edelgasen einstellt. Dann werden zusätzlich bis 20 bar Sauerstoffpartialdruck aufgedrückt und der während der Reaktion verbrauchte Sauerstoff kontinuierlich oder diskontinuierlich ersetzt.

Weiterhin ist es vorteilhaft, während des Ablaufs der Reaktion für eine gute Durchmischung der Reaktionskomponenten zu sorgen, beispielsweise durch intensives Rühren oder Verwendung eines Begasungsrührers.

Das erfindungsgemäße Verfahren kann innerhalb der angegebenen Parameter so durchgeführt werden, daß der Umsatz von 2,3-Dimethylbutenen beispielsweise 5 bis 99,9 % beträgt. Vorzugsweise beträgt dieser Umsatz am Ende der Reaktion 15 bis 95 %, besonders bevorzugt 20 bis 90 %.

Es ist vorteilhaft, die Oxidation der 2,3-Dimethylbutene ohne Zugabe von Wasser durchzuführen. Geringe Wassermengen, von z. B. bis zu 0,009 Gew.-%, die in den Einsatzprodukten vorhanden sind oder während der Reaktion gebildet werden, beeinträchtigen nicht die Bildung von Tetramethyloxiran.

Das erfindungsgemäße Verfahren kann sowohl in der Flüssig- als auch in der Gasphase durchgeführt werden. Vorzugsweise wird es in der Flüssigphase durchgeführt.

Eine technisch bevorzugte Durchführungsform des erfindungsgemäßen Verfahrens wird beispielsweise so durch geführt, daß man die 2,3-Dimethylbutene bei Temperaturen von 80 bis 140 °C, bei Drucken von 4 bis 70 bar, bei Reaktionszeiten von 10 bis 50 Minuten mit Sauerstoff oder sauerstoffhaltigen Gasen ohne Katalysator umsetzt, so daß der 2,3-Dimethylbuten-Umsatz 20 bis 85 % beträgt. Die Zusammensetzung eines so erhaltenen Reaktionsgemisches kann beispielsweise 5 bis 15 Gew.-% 2,3-Dimethylbutene, 55 bis 65 Gew.-% Tetramethyloxiran, 3 bis 7 Gew.-% 2,3-Dimethyl-1-buten-3-ol, 0,1 bis 1,5 Gew.-% 2,3-Dimethylbutan-2-diol, 3,0 bis 14,5 Gew.-% von niedriger als 2,3-Dimethylbutene und 3 bis 11,5 Gew.-% von höher als 2,3-Dimethylbutan-2,3-diol siedenden Verbindungen.

Bei 2,3-Dimethylbuten-Umsätzen von bis zu 90 % erzielt man nach dem erfindungsgemäßen Verfahren Tetramethyloxiran-Selektivitäten von beispielsweise 70 bis 80 %. Das erfindungsgemäße Verfahren kann auch mit 2,3-Dimethylbuten-Umsätzen von größer als 90 % durchgeführt werden, doch nimmt die Tetramethyloxiran-Selektivität bei höheren Umsätzen unter Umständen etwas ab.

Die Durchführung des erfindungsgemäßen Verfahrens kann diskontinuierlich oder kontinuierlich in den für Umsetzungen dieser Art üblichen Vorrichtungen erfolgen. Beispielsweise sind Rührkessel, Siedereaktoren, Röhrenreaktoren, Schlaufenreaktoren, Schleifenreaktoren, Dünnschichtreaktoren, Rührkesselkaskaden und Blasensäulen geeignet.

Als Werkstoffe für die Apparate zur Durchführung des erfindungsgemäßen Verfahrens können verschiedene Materialien verwendet werden. Beispielsweise sind Glas, Edelstahl, Nickellegierungen, Zirkon, Tantal und emaillierte Materialien geeignet.

Die Reaktionswärme kann auf verschiedene Weise abgeführt werden, beispielsweise durch innen- oder außenliegende Kühler oder durch Sieden unter Rückfluß, z. B. in Siedereaktoren.

Die 2,3-Dimethylbutene können auf verschiedene Art in die für die Umsetzung vorgesehene Vorrichtung eingebracht werden. Es ist möglich, die 2,3-Dimethylbutene an verschiedenen Stellen der Reaktionszone zuzuführen. Bei Verwendung mehrerer als Kaskade angeordneter Reaktoren kann es vorteilhaft sein, die 2,3-Dimethylbutene ausschließlich dem ersten Reaktor zuzuführen. Man kann ihre Zugabe aber auch auf verschiedene oder alle Reaktoren aufteilen.

Bei Verwendung mehrerer als Kaskade angeordneter Reaktoren kann man Sauerstoff oder Sauerstoff enthaltende Gasgemische und/oder 2,3-Dimethylbutene getrennt oder zusammen in jeden oder nur einige der Kessel der Kaskade geben.

Die Aufarbeitung der nach Durchführung des erfindungsgemäßen Verfahrens vorliegenden Reaktionsgemische und die Abtrennung des erhaltenen Tetramethyloxirans kann auf verschiedene Weise erfolgen. Wenn bei Temperaturen oberhalb des Siedepunktes (bei Normaldruck) der 2,3-Dimethylbutene und bei erhöhtem Druck gearbeitet wurde, kann es zweckmäßig sein, zunächst auf Temperaturen unterhalb des Siedepunktes (bei Normaldruck) der 2,3-Dimethylbutene abzukühlen, den Druck zu entspannen und dann das Tetramethyloxiran abzutrennen. Es ist unter Umständen vorteilhaft, die Druckentspannung unter Einleitung von inerten Gasen, wie Stickstoff, Kohlendioxid oder Edelgasen, vorzunehmen, um die Bildung explosionsfähiger Gasgemische auszuschließen. Das bei der Druckentspannung freiwerdende Gasgemisch kann, gegebenenfalls nach Reinigung und Anreicherung mit Sauerstoff, wieder in die Reaktion zurückgeführt werden.

Aus dem nach der Reaktion enthaltenen Reaktionsgemisch können das entstandene Tetramethyloxiran, die nicht umgesetzten 2,3-Dimethylbutene und die niedriger als 2,3-Dimethylbutene siedenden Nebenprodukte abgetrennt werden Diese Abtrennung kann gemeinsam oder nacheinander durchgeführt werden, beispielsweise durch Destillation und/oder Extraktion. Bei dieser Abtrennung können kleine Mengen Wasser, die evtl. während der Reaktion entstanden oder mit den Einsatzprodukten eingebracht worden sind, ganz oder teilweise entfernt werden.

Im Falle der gemeinsamen Abtrennung von Oxiran, nicht umgesetzten 2,3-Dimethylbutenen und gegebenenfalls weiteren Reaktionsprodukten kann eine Auftrennung dieses Gemisches in seine Komponenten beispielsweise durch Destillation, Extraktion, Absorption, Adsorption oder Extraktivdestillation erfolgen. Die nicht umgesetzten 2,3-Dimethylbutene können, gegebenenfalls nach vorheriger Reinigung und nach Zugabe der bei der Reaktion verbrauchten Menge an 2,3-Dimethylbutenen, erneut der Reaktionszone zugeführt werden.

Das so erhaltene Tetramethyloxiran kann bereits als solches für viele Zwecke verwendet werden, beispielsweise für die Herstellung von Polyethern, Polyurethanen, Epoxidharzen, Detergentien, Glykolen und einer Vielzahl von organischen Zwischenprodukten (siehe beispielsweise US-PS 2 412 136).

Das erfindungsgemäße Verfahren ermöglicht eine einfache und wirtschaftliche Herstellung von Tetramethyloxiran. Die erzielbaren Selektivitäten sind hoch, die Verwendung von im Vergleich zu Sauerstoff oder Luft teurem Wasserstoffperoxid wird vermieden, Katalysatoren und Lösungsmittel werden nicht benötigt und die Reaktionszeiten sind kurz.

Es ist ausgesprochen überraschend, das mit dem erfindungsgemäßen Verfahren diese Vorteile realisierbar sind, denn im Hinblick auf die Literaturstelle J. Org. Chem. 37, 2881 bis 2884 (1972) und US 4 182 722 konnte nicht erwartet werden, daß Tetramethyloxiran ohne Katalysatoren und Lösungsmittel und bei kurzen Reaktionszeiten hergestellt werden kann.

Das erfindungsgemäße Verfahren wird durch die folgenden Beispiele erläutert, ohne es darauf einzuschränken. Sämtliche Prozentangaben stellen, soweit nicht anderes gesagt wird, Gewichtsprozente dar.

Beispiele

Beispiel 1

In einem Edelstahl-Autoklaven, der mit einem Begasungsrührer, einem Thermometer und einem Ventil versehen war, wurden 255 g eines Gemisches, bestehend aus 98 % 2,3-Dimethyl-2-buten, 1 % 2,3-Dimethyl-1-buten und 1 % n-Hexan gegeben. Der Autoklav wurde unter Rühren (400 U/Min.) auf 100 °C erwärmt. Unter weiterem Rühren wurden fünf Minuten lang 28 bar Luft aufgedrück und noch fünf Minuten bei 100 °C nachgerührt. Dann wurde der Autoklav auf Raumtemperatur unter Rühren abgekühlt und die Reaktionsprodukte analysiert. Die Auswertung ergab 8,1 % 2,3-Dimethylbuten-Umsatz mit 76 % Selektivität bezogen auf hergestelltes Tetramethyloxiran. Weiterhin wurden gefunden 2,3-Dimethyl-1-buten-3-ol mit 18,8 % Selektivität und 2,3-Dimethyl-2,3-butadien mit 3,3 % Selektivität.

Beispiel 2

Es wurde verfahren wie in Beispiel 1, jedoch wurde der Autoklav auf 117 °C erwärmt. Unter Rühren (1 000 U/min.) wurde 15 Minuten lang 6,5 bar Sauerstoff aufgedrückt. Dann wurde der Autoklav auf Raumtemperatur abgekühlt und die Reaktionsprodukte analysiert. Die Auswertung ergab 40,3 % 2,3-Dimethylbuten-Umsatz mit 71 % Selektivität, bezogen auf hergestelltes Tetramethyloxiran. Weiterhin wurden gefunden 2,3-Dimethyl-1-buten-3-ol mit 15,5 % Selektivität, 2,3-Dimethylbutan-2-ol mit 0,5 Selektivität, 3,3-Dimethylbutan-2-on mit 0,4 % Selektivität und 2,3-Dimethylbutadien mit 0,3 % Selektivität.

Beispiel 3

In den im Beispiel 1 beschriebenen Autoklaven wurden 255 g eines Gemisches, bestehend aus 80 % 2,3-Dimethyl-2-buten, 15 % 2,3-Dimethyl-1-buten, 2 % 2,3-Dimethylbutan und 3 % n-Hexan, gegeben. Nach Aufheizen auf 87 °C wurden unter Rühren (1 400 U/Min.) 20 bar eines aus 35 % Sauerstoff und 65 % Stickstoff bestehenden Gasgemisches und nach weiteren 5 Minuten 25 Minuten lang 3 bar reiner Sauerstoff aufgedrückt. Dann wurde der Autoklav auf Raumtemperatur abgekühlt und die Reaktionsprodukte analysiert. Die Auswertung ergab 83,1 % 2,3-Dimethylbuten-Umsatz mit 72 % Selektivität, bezogen auf hergestelltes Tetramethyloxiran. Weiterhin wurden gefunden 2,3-Dimethyl-1-buten-3-ol mit 16,1 % Selektivität, 2,3-Dimethyl-2,3-butandiol mit 1,2 % Selektivität, 2,3-Dimethylbutan-2-ol mit 0,6 % Selektivität, 3,3-Dimethylbutan-2-on mit 0,5 % Selektivität und 3,3-Dimethylbutan-2-ol mit 0,3 % Selektivität.

Beispiel 4

Es wurde verfahren wie in Beispiel 3, jedoch wurde das erhaltene Reaktionsgemisch durch Destillation in die Komponenten aufgetrennt, wobei die wiedergewonnenen 2,3-Dimethylbutene erneut in die Reaktion eingesetzt wurden. Die Versuchsauswertung ergab 85,6 % 2,3-Dimethylbuten-Umsatz mit 69,2 % Selektivität, bezogen auf isoliertes Tetramethyloxiran.

Beispiel 5

In den im Beispiel 1 beschriebenen Autoklaven wurden 208 g eines Gemisches, bestehend aus 95 % 2,3-Dimethyl-2-buten, 3 % 2,3-Dimethyl-1-buten, 1,5 % 2,3-Dimethylbutan und 0,5 % n-Hexan, gegeben. Nach Erwärmen auf 128 °C wurde unter Rühren (2 000 U/Min.) 20 Minuten lang 5,3 bar Sauerstoff aufgedrückt. Dann wurde der Autoklav auf Raumtemperatur abgekühlt. Die Versuchsauswertung ergab 65,3 % 2,3-Dimethylbuten-Umsatz mit 71,3 % Selektivität, bezogen auf hergestelltes Tetramethyloxiran. Weiterhin wurden gefunden 2,3-Dimethyl-1-buten-3-ol mit 13,3 % Selektivität, 2,3-Dimethylbutan-2,3-diol mit 5,9 % Selektivität, 2,3-Dimethylbutan-2-ol mit 2,3 % Selektivität, 2,3-Dimethylbutan-2-on mit 2,2 % Selektivität und 3,3-Dimethylbutan-2-ol mit 1,7 % Selektivität.

Beispiel 6

In eine aus drei mit je einem Begasungsrührer (1 500 U/Min.) gerührten Druckreaktoren von je 170 ml Inhalt bestehende Kaskade wurden 930 g/h eines Gemisches, bestehend aus 97 % 2,3-Dimethyl-2-buten, 1,3 % 2,3-Dimethyl-1-buten, 0,8 % 2,3-Dimethylbutan und 0,9 % n-Hexan, eingegeben. Der erste Reaktor der Kaskade wurde bei 105 °C betrieben, der zweite bei 100 °C und der dritte bei 95 °C. Nach Erreichen der vorgegebenen Reaktionstemperaturen wurde auf jeden Reaktor über ein Ventil soviel Sauerstoff aufgedrückt, daß der Gesamtdruck in der Kaskade 7,5 bar betrug. Das Reaktionsgemisch wurde nach der Kaskade druckentspannt unter Zusatz von Stickstoff, um explosionsfähige Gasgemische auszuschließen. Danach wurde das Reaktionsgemisch auf 60 °C abgekühlt und einer Siebbodenkolonne zur Abtrennung von niedriger als 2,3-Dimethylbutene siedenden Verbindungen sowie von nichtumgesetzten 2,3-Dimethylbutenen zugeführt. Nach Zusatz von frischem 2,3-Dimethylbuten-Gemisch wurden die zurückgewonnenen 2,3-Dimethylbutene in den ersten Reaktor der Kaskade zurückgeführt. In einer zweiten Kolonne wurden das gebildete Tetramethyloxiran und bei Normaldruck niedriger als 180 °C siedende Bestandteile des verbleibenden Reaktionsgemisches im Vakuum destillativ abgetrennt.

Die analytische Auswertung ergab 72,5 % 2,3-Dimethylbuten-Umsatz mit einer Selektivität von 73 %, bezogen auf hergestelltes Tetramethyloxiran. Man erhielt pro Stunde 293,5 g Tetramethyloxiran.

## Patentansprüche

1. Verfahren zur Herstellung von Tetramethyloxiran durch Umsetzung von 2,3-Dimethylbutenen, die bis zu 2 % 2,3-Dimethylbutan und bis zu 3 % n-Hexan enthalten können, mit Sauerstoff oder einem Sauerstoff enthaltenden Gas bei Temperaturen von 40 bis 180 °C und Verweilzeiten von 5 bis 150 Minuten ohne Zusatz von Katalysatoren, dadurch gekennzeichnet, daß man ohne Zusatz von inerten Lösungsmitteln arbeitet und das erhaltene Tetramethyloxiran abtrennt.

2. Verfahren nach Anspruch 1, dadurch gekennzeichnet, daß man die Umsetzung bei Drücken im Bereich 0,9 bis 90 bar durchführt.

3. Verfahren nach Anspruch 1 und 2, dadurch gekennzeichnet, daß man 2,3-Dimethyl-2-buten, 2,3-Dimethyl-1-buten oder beliebige Gemische aus 2,3-Dimethyl-2-buten und 2,3-Dimethyl-1-buten einsetzt.

4. Verfahren nach Anspruch 1 bis 3, dadurch gekennzeichnet, daß man Luft, Sauerstoff enthaltende Gasgemische, die über 50 % Sauerstoff enthalten oder reinen Sauerstoff einsetzt.

5. Verfahren nach Anspruch 1 bis 4, dadurch gekennzeichnet, daß man den Sauerstoff in mehreren Teilmengen nacheinander zugibt.

6. Verfahren nach Anspruch 1 bis 5, dadurch gekennzeichnet, daß man das erhaltene Reaktionsgemisch nach Druckentspannung durch Destillation in die Komponenten auftrennt und nicht-umgesetzte 2,3-Dimethylbutene ganz oder teilweise in die Reaktion zurückführt.

7. Verfahren nach Anspruch 1 bis 5, dadurch gekennzeichnet, daß man das erhaltene Reaktionsgemisch nach Druckentspannung durch Extraktion in die Komponenten auftrennt und nicht-umgesetzte 2,3-Dimethylbutene ganz oder teilweise in die Reaktion zurückführt.

8. Verfahren nach Anspruch 1 bis 5, dadurch gekennzeichnet, daß man das erhaltene Reaktionsgemisch nach Druckentspannung durch Absorption in die Komponenten auftrennt und nicht-umgesetzte 2,3-Dimethylbutene ganz oder teilweise in die Reaktion zurückführt.

9. Verfahren nach Anspruch 1 bis 5, dadurch gekennzeichnet, daß man das erhaltene Reaktionsgemisch nach Druckentspannung durch Adsorption in die Komponenten auftrennt und nicht-umgesetzte 2,3-Dimethylbutene ganz oder teilweise in die Reaktion zurückführt.

10. Verfahren nach Anspruch 1 bis 5, dadurch gekennzeichnet, daß man das erhaltene Reaktionsgemisch nach Druckentspannung durch Extraktivdestillation in die Komponenten auftrennt und nichtumgesetzte 2,3-Dimethylbutene ganz oder teilweise in die Reaktion zurückführt.

## Claims

1. Process for preparing tetramethyloxirane by reacting 2,3-dimethylbutenes which may contain up to 2 % of 2,3-dimethylbutane and up to 3 % of n-hexane with oxygen or an oxygen-containing gas at temperatures of 40 to 180 °C and residence times of 5 to 150 minutes without the addition of catalysts, characterised in that the reaction is carried out without the addition of inert solvents and the tetramethyloxirane obtained is separated off.

2. Process according to Claim 1, characterised in that the reaction is carried out under pressures in the range 0.9 to 90 bar.

3. Process according to Claim 1 and 2, characterised in that 2,3-dimethyl-2-butene, 2,3-dimethyl-1-butene or any desired mixtures of 2,3-dimethyl-2-butene and 2,3-dimethyl-1-butene are used.

4. Process according to Claim 1 to 3, characterised in that air, oxygen-containing gas mixtures which contain more than 50 % of oxygen or pure oxygen are used.

5. Process according to Claim 1 to 4, characterised in that the oxygen is added in several successive portions.

6. Process according to Claim 1 to 5, characterised in that after the pressure has been let down the reaction mixture obtained is separated into its components by distillation and unconverted 2,3-dimethylbutene is completely or partially returned to the reaction.

7. Process according to Claim 1 to 5, characterised in that after the pressure has been let down the reaction mixture obtained is separated into its components by extraction and unconverted 2,3-dimethylbutene is completely or partially returned to the reaction.

8. Process according to Claim 1 to 5, characterised in that after the pressure has been let down the reaction mixture obtained is separated into its components by absorption and unconverted 2,3-dimethylbutene is completely or partially returned to the reaction.

9. Process according to Claim 1 to 5, characterised in that after the pressure has been let down the reaction mixture obtained is separated into its components by adsorption and unconverted 2,3-dimethylbutene is completely or partially returned to the reaction.

10. Process according to Claim 1 to 5, characterised in that after the pressure has been let down the reaction mixture obtained is separated into its components by extractive distillation and unconverted 2,3-dimethylbutene is completely or partially returned to the reaction.

## Revendications

1. Procédé de préparation de tétraméthyloxirane par réaction de diméthyl-2,3 butènes pouvant contenir jusqu'à 2 % de diméthyl-2,3 butane et jusqu'à 3 % de n-hexane, avec de l'oxygène ou un gaz contenant de l'oxygène à des températures de 40 à 180 °C et avec des temps de séjour de 5 à 150 minutes sans addition de catalyseurs, caractérisé en ce qu'on opère sans addition de solvants inertes et sépare le tétraméthyloxirane obtenu.

2. Procédé selon la revendication 1, caractérisé en ce qu'on effectue la réaction sous des pressions dans le domaine de 0,9 à 90 bars.

3. Procédé selon les revendications 1 et 2, caractérisé en ce qu'on met en œuvre du diméthyl-2,3 butène-2, du diméthyl-2,3 butène-1 ou des mélanges au choix de diméthyl-2,3 butène-2 et de diméthyl-2,3 butène-1.

4. Procédé selon les revendications 1 à 3, caractérisé en ce qu'on utilise de l'air, des mélanges gazeux contenant de l'oxygène qui renferment plus de 50 % d'oxygène ou de l'oxygène pur.

5. Procédé selon les revendications 1 à 4, caractérisé en ce qu'on introduit l'oxygène successivement en plusieurs fractions.

6. Procédé selon les revendications 1 à 5, caractérisé en ce que, après détente, on sépare le mélange réactionnel résultant en ses constituants par distillation et recycle les diméthyl-2,3 butènes non transformés en partie ou en totalité dans la réaction.

7. Procédé selon les revendications 1 à 5, caractérisé en ce que, après détente, on sépare le mélange réactionnel résultant en ses constituants par extraction et recycle les diméthyl-2,3 butènes non transformés en partie ou en totalité dans la réaction.

8. Procédé selon les revendications 1 à 5, caractérisé en ce que, après détente, on sépare le mélange réactionnel résultant en ses constituants par absorption et recycle les diméthyl-2,3 butènes non transformés en partie ou en totalité dans la réaction.

9. Procédé selon les revendications 1 à 5, caractérisé en ce que, après détente, on sépare le mélange réactionnel résultant en ses constituants par adsorption et recycle les diméthyl-2,3 butènes non transformés en partie ou en totalité dans la réaction.

10. Procédé selon les revendications 1 à 5, caractérisé en ce que, après détente, on sépare le mélange réactionnel résultant en ses constituants par distillation extractive et recycle les diméthyl-2,3 butènes non transformés en partie ou en totalité dans la réaction.